Europäisches Patentamt

European Patent Office

Office européen des brevets

(11) Veröffentlichungsnummer: **0 054 679**
**B1**

(12) **EUROPÄISCHE PATENTSCHRIFT**

(45) Veröffentlichungstag der Patentschrift:
**14.08.85**

(21) Anmeldenummer: **81108795.6**

(22) Anmeldetag: **23.10.81**

(51) Int. Cl.⁴: **G 01 N 33/52,** C 12 Q 1/00,
**G 01 N** 31/22

(54) **Verwendung von Reagenzstreifen zur Herstellung von Reagenzlösungen.**

(30) Priorität: **23.12.80 DE 3048799**

(43) Veröffentlichungstag der Anmeldung:
**30.06.82 Patentblatt 82/26**

(45) Bekanntmachung des Hinweises auf die Patenterteilung:
**14.08.85 Patentblatt 85/33**

(84) Benannte Vertragsstaaten:
**AT BE CH DE FR GB IT LI LU NL SE**

(56) Entgegenhaltungen:
**EP - A - 0 005 519**
**DE - A - 2 301 999**
**DE - A - 2 655 977**
**DE - A - 2 739 008**
**DE - B - 2 118 455**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Lange, Hans-Rudolf, Danzigerstrasse 3, D-6840 Lampertheim (DE)**
Erfinder: **Geisler, Edda, Feldbergstrasse 58, D-6800 Mannheim 23 (DE)**
Erfinder: **Werner, Wolfgang, Dr. rer.Nat., Meissener Weg 39, D-6800 Mannheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue Verwendung von Reagenzstreifen zur Herstellung von Reagenzlösungen zur chemischen Analyse von Bestandteilen von Flüssigkeiten.

Da eine direkte physikalische oder physikochemische Analyse nur in Sonderfällen möglich ist, bildet die chemische Analyse, d.h. die Zugabe von geeigneten Reagentien und die Bestimmung der dadurch bewirkten Reaktion immer noch die wichtigste, qualitative und quantitative Untersuchungsmethode für Bestandteile von Flüssigkeiten. Die Reagentien werden dabei häufig im Überschuss zugegeben, müssen jedoch bei anderen Analysenverfahren, wie einer kinetischen Bestimmung oder der Massanalyse, in genauer Dosierung zugefügt werden. Wegen der guten Dosierbarkeit werden feste Reagentien vor der Zugabe normalerweise in einem geeigneten Lösungsmittel aufgelöst und ein aliquoter Teil der Lösung für die Analyse benutzt. Um dem Benutzer das, insbesondere bei mehreren Reagentien, mühsame Abwiegen und Lösen zu ersparen, sind solche Reagenzlösungen in fertiger Form bereits seit langem im Handel erhältlich. Reagentien, die in Lösung miteinander unverträglich sind, müssen dabei getrennt gelöst und kurz vor der Benutzung vereinigt werden. Reagentien, die in Lösung instabil sind, werden üblicherweise als «Reagenztablette» unter Zusatz geeigneter Tablettierhilfsmittel, oder als Lyophilisat in Ampullen angeboten, die kurz vor der Benutzung durch Zugabe eines Lösungsmittels zur Reagenzlösung rekonstituiert werden können. Reagenztabletten haben dabei den Nachteil, dass sie entweder bei Bearbeitung und Lagerung durch Abrieb ihre Masse und damit die Menge an Reagentien verändern können, oder so hart gepresst werden müssen, dass ihre Wiederauflösung Schwierigkeiten bereitet. Eine Störung der Analyse durch die Tablettierhilfsstoffe, insbesondere Gleitmittel und Formtrennmittel, schränkt ihre allgemeine Verwendbarkeit ein.

Die Lyophilisierung von Reagenzlösungen in Ampullen, ist von der Rekonstituierbarkeit her optimal und gestattet auch miteinander «unverträgliche» Reagentien als «Schichtenlyophilisat» übereinander einzufrieren und zu trocknen. Das Verfahren ist jedoch sehr aufwendig und teuer.

In der DE-OS 2 301 999 wurde vorgeschlagen, in Lösung instabile Substanzen dergestallt zu stabilisieren, dass man einen saugfähigen Träger, beispielsweise Filterpapier, mit der Reagenzlösung tränkt, trocknet oder lyophilisiert und Stükke geeigneter Grösse und damit geeigneter Regenzmenge auf einem als «Mischstäbchen» dienenden Handgriff, beispielsweise aus Kunststoff, aufklebt. Durch Rühren mit diesem Mischstäbchen in einer entsprechenden Menge eines Lösungsmittels beziehungsweise der Testflüssigkeit werden die Reagentien wieder eluiert und das fertige Analysengemisch erhalten. Obwohl diese Technik im Prinzip brauchbar ist, zeigt sich, dass von so hergestellten Mischstäbchen nur sehr gut lösliche Stoffe rasch und vollständig abgelöst werden, die üblichen Reagentien aber unvollständig eluiert werden. Abgesehen davon, dass man dadurch unnötig grosse Mengen von Reagentien auf den Träger imprägnieren muss, ist die eluierte Menge auch noch von den Bedingungen der Elution (Zeit, Rührgeschwindigkeit, Temperatur, Viskosität etc.) abhängig, so dass eine exakte Konzentration nicht garantiert werden kann.

In der DE-A 2 739 008 sind weiterhin Testvorrichtungen beschrieben, in denen die Oberfläche des Reagenzträgers mit quellförmigen Polymerpartikeln bedeckt ist, die einen Überschuss der Testflüssigkeit aufsaugen und ein Auswaschen der Reagenzien verhindern sollen.

Es stellte sich deshalb die Aufgabe, Reagenzstreifen bereitzustellen, bei deren Verwendung sich die Reagenzien rasch, reproduzierbar und möglichst vollständig ablösen lassen.

Die Erfindung betrifft die Verwendung von vom Aufbau her an sich bekannten Reagenzstreifen zur Herstellung von Reagenzlösungen, wie in den Ansprüchen definiert.

Das Einsiegeln von reagenzhaltigen, saugfähigen Trägern zwischen einem Kunststoffgriff und einem dünnen Netz ist bereits in der DE-AS 2 118 455 beschrieben, jedoch wird bei den dort beschriebenen ca. 6×6 mm grossen Testpapieren selbst im Harnstrahl kein «Auswascheffekt» beobachtet (Spalte 3, 6–16). Es ist deshalb als überraschend anzusehen, dass durch die in den Ansprüchen gekennzeichneten zusätzlichen Massnahmen von einem solchen Reagenzstreifen durch kurzes Rühren in einem Lösungsmittel die Reagenzien praktisch quantitativ eluiert werden, während sie von einem entsprechenden aufgeklebten Träger (gemäss der DE-OS 2 301 999), dessen Oberseite für den Zutritt des Lösungsmittels völlig frei liegt, nur unvollkommen abgelöst werden.

Vorrichtungen, die den erfindungsgemäss verwendeten Reagenzstreifen im Aufbau ähneln, sind für einen völlig anderen Zweck in der EP-A 5 519 beschrieben. Bei diesem zum Nachweis von Ammoniak der Ammoniak-bildenden Stoffen, wird eine definierte Menge der Testlösung durch ein Abdecknetz auf einen Reagenzträger gegeben, in dem gasförmiges Ammoniak freigesetzt wird, welches durch ein hydrophobes Zwischennetz, welches das Gas durchlässt, aber die Flüssigkeit zurückhält, in eine darunterliegende 2. Reagenzschicht diffundiert und eine Verfärbung bewirkt. Zur Identifikation werden das Abdecknetz, die 1. Reagenzschicht und das Zwischennetz entfernt. Eine Auswaschung der Reagenzien ist nicht vorgesehen, so dass daraus kein Hinweis auf die vorliegende Erfindung entnommen werden kann.

Die Ablösungsgeschwindigkeit lässt sich noch steigern, wenn die für die Aufnahme grösserer Reagenzmengen notwendig werdenden dickeren Papiere in mehrere Lagen dünner Papiere unterteilt werden, wodurch eine zusätzliche Querdurchströmung bewirkt wird. Solche getrennten Papierlagen können auch dazu dienen, unverträgliche Reagenzien physikalisch voneinander zu trennen.

Darüberhinaus kann die Durchströmung und damit die Elutionsgeschwindigkeit verbessert werden, wenn zwischen solche Papierlagen ein weiteres Kunststoffnetz (Zwischennetz) mit eingeklemmt wird. Für den Fall, dass in verschiedenen Papierlagen unterschiedliche Reagentien verwendet werden, wird durch solche Zwischennetze eine eventuell verbliebene Interaktion mit Sicherheit ausgeschaltet.

Weiterhin ist es, wenn der saugfähige Träger aus mehreren Lagen besteht, auch möglich, eine dieser Lagen zwischen dem Unterlegnetz und dem Handgriff vorzusehen.

Obwohl auch Naturfaser- oder Metalldrahtnetze vom Prinzip her brauchbar sind, werden wegen der einfachen Verarbeitung und des günstigeren Preises erfindungsgemäss bevorzugt Kunststoffnetze, beispielsweise aus Polyamid, Polyester, PVC etc., zum Einsiegeln und als Zwischen- und Unterlegnetze verwendet. Maschenweiten von 50–250 µm, vorzugsweise 100–150 µm haben sich für die Abdecknetze, Maschenweiten von 80–250 µ vorzugsweise 100–200 µm für die Unterlegnetze bewährt. Die Fadenstärken liegen üblicherweise zwischen 10 und 50 µm. Gewebte oder gewirkte Netze kommen gleicherweise infrage, wobei die Einzelfäden an den Kreuzungspunkten zusätzlich miteinander verschweisst oder verklebt sein können.

Als saugfähige Träger kommen bevorzugt Papiere oder Vliese aus Baumwolle, Zellstoff, regenerierter Cellulose, Kunstfasern beispielsweise aus Polyamid, Polyester etc. oder Gemische davon infrage, wobei Materialien, die eine chemische Bindung oder Reaktion oder feste Komplexe mit den Reagentien eingehen, natürlich ausgeschlossen sind. Wegen der leichten Ablösbarkeit und guten Saugfähigkeit, sowie ihrer guten Verarbeitbarkeit auch nach Imprägnierung mit viel Reagenz, werden Polyamid- und Polyamid/Cellulose-Papiere besonders bevorzugt.

Die erfindungsgemäss verwendeten Reagenzstreifen erlauben auch, empfindliche feste Reagenzien in stabiler Form zu lagern und rasch zur Reagenzlösung zu rekonstituieren. Über die Dikke und Grösse des verwendeten Trägers, sowie die Konzentration der zur Imprägnierung verwendeten Reagenzlösung lässt sich die Reagenzkonzentration in der fertigen Lösung in weiten Bereichen steuern. Bei grösserem Reagenzbedarf lassen sich auch mehrere Papiere über oder nebeneinander an einem Handgriff befestigen. Die Aufteilung der Reagentien auf mehrere Papierstücke erlaubt es, entsprechende Reagenzpapiere für unterschiedliche Teste, beispielsweise Puffermischungen, zusammen zu fertigen und beim Einsiegeln in geeigneter Zusammensetzung zu kombinieren.

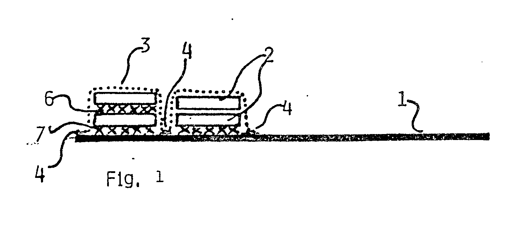

Der Aufbau der erfindungsgemäss verwendeten Reagenzstreifen ist beispielhaft in der beigefügten Figur 1 gezeigt.

Die Figur 1 zeigt in einer Seitenansicht einen Reagenzstreifen, bei dem auf einem Handgriff (1) zwei getrennte, doppellagige, reagenzhaltige, saugfähige Träger (2) mittels eines Abdecknetzes (3) über die Haftstellen (4) verbunden sind. Die Reagenzpapiere der einen Doppellage sind dabei durch ein Zwischennetz (6) getrennt und beide Doppellagen durch ein Unterlegnetz (7) vom Handgriff (1) abgehoben.

Die technische Herstellung der Reagenzstreifen erfolgt zweckmässig durch kontinuierliches Zusammensiegeln oder -kleben langer Kunststoff-, Reagenzpapier- und Netzbahnen in einer bekannten Siegelvorrichtung. Das Auseinanderschneiden der erhaltenen Bänder in Querrichtung zu Streifen der gewünschten Breite kann in Analogie zu der in der DE-AS 2 118 455 beschriebenen Weise erfolgen. Für die in den folgenden Beispielen beschriebenen Teste wurden die Reagenzstreifen entsprechend hergestellt, obwohl sich Labormuster und Kleinserien natürlich auch durch Befeuchten von vorher hergestellten «leeren» Reagenzstreifen mit einer abgemessenen Menge einer Reagenzlösung und anschliessendem Trocknen herstellen lassen.

Die Beispiele dienen zur Erläuterung der Erfindung und sollen diese nicht beschränken.

Beispiel 1

Reagenzstreifen für die Bestimmung von Triglyceriden im Serum

In einem geeigneten Glasgefäss, in welchem 13 ml einer über 1 Jahr bei Raumtemperatur stabilen Pufferlösung von pH 8,1 vorgelegt wurde und die 0,1 mol/l Glycylglycin, 0,16 mol/l $NH_4Cl$, 1 mmol/l Natriumcholat, 0,2% Detergenz und 1 g/l Natriumazid enthält, wird ein Reagenzstreifen von ca. 100 mm Länge und 10 mm Breite eingestellt, der in zwei Bezirken je 2 übereinanderliegende Reagenzpapiere der Flächen 10×15 mm in eingesiegelter Form enthält. Zur Beschleunigung der Substanzablösung in den Reagenzpapierbezirken ist zusätzlich je 1 Gewebe mit ca. 250 µm Maschenweite von der Fläche 10×15 mm unterlegt. Der am unteren Ende des Reagenzstreifens angeklemmte Reagenzbezirk enthält auf dem einen Reagenzträger 13 mg NAD und auf dem darüberliegenden Reagenzträger 3,9 mg 3-(4,5-Dimethylthiazolyl-2)-2,5-diphenyltetrazoliumbromid (MTT).

Der danebenliegende Reagenzbezirk enthält 2 Reagenzträger mit je 45 U Glycerindehydrogenase, 6 U Diaphorase, 13 U Cholesterinesterase.

Der Reagenzstreifen wird 5 Sekunden intensiv bewegt, anschliessend wird 5 Minuten lang stehen gelassen, nochmals 5 Sekunden bewegt und der eluierte Reagenzträger verworfen.

Es entsteht eine Reaktionslösung mit folgender Zusammensetzung:

| | |
|---|---|
| Glycylglycin | 0,1 mol/l |
| $NH_4Cl$ | 0,16 mol/l |
| Na-Cholat | 1 mmol/l |
| Detergenz | 0,2% |
| MTT | 270 mg/l |
| NAD | 1000 mg/l |

Glycerindehydrogenase      6 KU/l
Diaphorase                 400 U/l
Cholesterinesterase        900 U/l

Der Triglyceridgehalt im Serum wird ermittelt, indem zu 2 ml dieser Reaktionslösung 0,020 ml Probe hinzugegeben und nach 30 Minuten Inkubation bei 20–25°C die Extinktion bei Hg 578 nm gegen einen Reagentienleerwert (RL) ermittelt wird. Die Konzentration (C) der Triglyceride wird berechnet nach

$$C[mg/100\ ml] = 498,5 \times (E - E_{RL})$$

**Patentansprüche**

1. Verwendung eines Reagenzstreifens, der aus einem Handgriff (1) besteht und an dessen unterem Teil ein mit einem Reagenz imprägnierter saugfähiger Träger (2) mit einem dünnen Netz (3), welches auf einander gegenüberliegenden Seiten des Trägers (2) auf dem Handgriff (1) angeklebt oder angesiegelt und zwischen dem saugfähigen Träger und dem Handgriff ein Unterlegnetz (7) eingeklemmt ist, zur Herstellung einer Reagenzlösung, wobei der Regenzstreifen in einem Lösungsmittel oder Diluens eluiert wird.

2. Verwendung von Reagenzstreifen gemäss Anspruch 1, dadurch gekennzeichnet, dass mehrere saugfähige Träger (2) nebeneinander oder sandwichartig übereinander auf dem Handgriff befestigt sind.

3. Verwendung von Regenzstreifen nach Anspruch 2, dadurch gekennzeichnet, dass die verschiedenen Träger (2) unterschiedliche Reagenzien tragen.

4. Verwendung von Reagenzstreifen gemäss Anspr. 1–3, dadurch gekennzeichnet, dass zwischen zwei übereinanderliegenden Trägern (2) ein Zwischennetz (6) eingeklemmt ist.

5. Verwendung von Reagenzstreifen gemäss Anspr. 1–3, dadurch gekennzeichnet, dass sich zwischen dem Unterlegnetz (7) und dem Handgriff (1) ein weiterer saugfähiger Träger (2) befindet.

6. Verwendung von Reagenzstreifen nach Ansprüchen 1–5, dadurch gekennzeichnet, dass der Handgriff (1) und die Netze (6) und (7) aus Kunststoff und der saugfähige Träger (2) aus Polyamidvlies oder Polyamid/Cellulose-Vlies besteht.

**Claims**

1. Use of a reagent strip which consists of a handle (1) and on its lower part an absorbent carrier (2) impregnated with a reagent is fixed with a thin mesh (3) which is stuck or sealed on the opposite-lying side of the carrier (2) on to the handle (1) and an underlying mesh (7) is inserted between the absorbent carrier and the handle, for the production of a reagent solution, whereby the reagent strip is eluted in a solvent or diluent.

2. Use of reagent strips according to claim 1, characterised in that several absorbent carriers (2) are fixed on to the handle side by side or sandwichlike on top of one another.

3. Use of reagent strips according to claim 2, characterised in that the different carriers (2) carry different reagents.

4. Use of reagent strips according to claims 1–3, characterised in that an intermediate mesh (6) is inserted between two overlying carriers (2).

5. Use of reagent strips according to claims 1–3, characterised in that a further absorbent carrier (2) is present between the underlying mesh (7) and the handle (1).

6. Use of reagent strips according to claims 1–5, characterised in that the handle (1) and the meshes (6) and (7) consist of synthetic resin and the absorbent carrier (2) consists of polyamide fleece or polyamide/cellulose fleece.

**Revendications**

1. Utilisation d'un bandelette réactive comportant une poignée (1) et à l'extrémité inférieure de celle-ci un support absorbant (2) imprégné d'un réactif, par serrage sous un filet (3) fin, ce dernier étant collé ou scellé sur la poignée (1) à des endroits situés de part et d'autre du support (2) et, serré entre le support absorbant et la poignée un filet intercalaire (7), pour la préparation d'une solution de réactifs, en éluant la bandelette réactive dans un solvant ou dans un diluant.

2. Utilisation de bandelettes réactives selon la revendication 1, caractérisée en ce que plusieurs supports absorbants (2) sont fixés côte à côte un superposés en sandwich sur la poignée.

3. Utilisation de bandelettes réactives selon la revendication 2, caractérisée en ce que les différents supports (2) comportent des réactifs différents.

4. Utilisation de bandelettes réactives selon les revendications 1 à 3, caractérisée en ce qu'un filet intercalaire (6) est serré entre deux supports (2) superposés.

5. Utilisation de bandelettes réactives selon les revendications 1 à 3, caractérisées en ce qu'entre le filet intercalaire (7) et la poignée (1) se trouve un support absorbant (2) supplémentaire.

6. Utilisation de bandelettes réactives selon les revendications 1 à 5, caractérisée en ce que la poignée (1) et les filets (6) et (7) sont en matière synthétique et le support absorbant (2) en voile de polyamide ou en voile de polyamide/cellulose.

Fig. 1